# EUROPEAN PATENT APPLICATION

(11) **EP 1 716 863 A1**
(43) Date of publication of application: **02.11.2006**
(21) Application number: 05706576.5
(22) Date of filing: 31.01.2005
(51) Int. Cl.: A61K 38/16, A61K 39/00, A61K 48/00, A61P 37/02

(54) **T CELL IMMUNE RESPONSE INHIBITOR**

(30) Priority: 20.02.2004 CN 200410039189
(71) Applicant: China Agricultural University, Haidian District, Beijing 100094 (CN)
(72) Inventor: WANG, Bin, Haidian District, Beijing 100094 (CN); YU, Qingling, Haidian District, Beijing 100094 (CN); JIN, Huali, Haidian District, Beijing 100094 (CN); KANG, Youmin, Haidian District, Beijing 100094 (CN)
(74) Representative: Bassil, Nicholas Charles
(86) International application number: PCT/CN2005/000136
(87) International publication number: WO 2005/079833

(57) **Abstract**

The invention discloses a T cell immune response inhibitor. The inventive T cell immune response inhibitor includes nucleic acid vaccine with regard to pathogen and the protein antigen expressed by this nucleic acid vaccine; or includes nucleic acid vaccine with regard to pathogen and the active peptide of the protein antigen expressed by this nucleic acid vaccine; or includes inactivated pathogen and nucleic acid vaccine with regard to this pathogen. The inventive T-cell immune response inhibitor may provoke the body produce normal specific antibody immune response, restrain specific cell-mediated immune response, especially Th1 type immune response, thereby may be effectively used for autoimmune disease, organ transplantation, hypersensitivity, T cell amount controlled treatment.

SEQUENCE LISTING

<110> China Agricultural University

Wang, Bin

Yu, Qingling

Kang, Youmin

Jin, Huali

<120> T-CELL IMMUNE RESPONSE INHIBITOR

<130> WANG0002-501

<140> PCT/CN2005/000136

<141> 2005-01-31

<150> CN200410039138 9.1

<151> 2004-02-20

<160> 16

<170> PatentIn version 3.3

<210> 1

<211> 30

<212> DNA

<213>  Artificial Primer

<220>
<223>  hepB primer

<400>  1
cggatccatt aagccatgca gtggaactcc                                    30

<210>  2
<211>  32
<212>  DNA
<213>  Artificial Primer

<220>
<223>  3 prime hepB primer

<400>  2
gtccttgggt atacatttga accccggatc ca                                 32

<210>  3
<211>  16
<212>  PRT
<213>  Artificial Peptide

<220>

<223> VP1 protein RGD peptide

<400> 3

Leu Arg Gly Asp Leu Gln Val Leu Ala Gln Lys Val Ala Arg Thr Leu
1               5                   10                  15

<210> 4
<211> 11
<212> PRT
<213> Artificial Peptide

<220>
<223> porcine reproductive and respiratory system virus E2 antigen

<400> 4

Cys Thr Ala Val Ser Pro Thr Thr Leu Arg Thr
1               5                   10

<210> 5

<211> 25

<212> DNA

<213> Artificial Primer

<220>

<223> HPRT 5' primer

<400> 5

gttggataca ggccagactt tgttg 25

<210> 6

<211> 22

<212> DNA

<213> Artificial Primer

<220>

<223> HPRT 3' primer

<400> 6

gagggtaggc tggcctatgg ct 22

<210> 7

<211> 19

<212> DNA
<213> Artificial Primer

<220>
<223> IL-2 5' primer

<400> 7
tccacttcaa gctctacag 19

<210> 8
<211> 21
<212> DNA
<213> Artificial Primer

<220>
<223> IL-2 3' primer

<400> 8
gagtcaaatc cagaacatgc c 21

<210> 9
<211> 22
<212> DNA

<213>  Artificial Primer

<220>
<223>  IFN-gamma 5' primer

<400>  9
cattgaaagc ctagaaagtc tg                                        22

<210>  10
<211>  23
<212>  DNA
<213>  Artificial Primer

<220>
<223>  IFN-gamma 3' primer

<400>  10
ctcatggaat gcatcctttt tcg                                       23

<210>  11
<211>  22
<212>  DNA
<213>  Artificial Primer

<220>

<223>  IL-4 5' primer

<400>  11

gaaagagacc ttgacacagc tg                                    22

<210>  12

<211>  22

<212>  DNA

<213>  Artificial Primer

<220>

<223>  IL-4 3' primer

<400>  12

gaactcttgc aggtaatcca gg                                    22

<210>  13

<211>  24

<212>  DNA

<213>  Artificial Primer

<220>

<223>  IL-10 5' primer

<400>  13

caagtttacc tggtagaagt gatg                                    24

<210>  14

<211>  30

<212>  DNA

<213>  Artificial Primer

<220>

<223>  IL-10 3' primer

<400>  14

tgtctaggtc ctggagtcca gcagactcaa                              30

<210>  15

<211>  32

<212>  DNA

<213>  Artificial Primer

<220>

<223>  VP1 5' primer

<400>  15
aagaattcgg aggtaccacc tctgcgggtg ag                                32

<210>  16
<211>  37
<212>  DNA
<213>  Artificial Primer

<220>
<223>  VP1 3' primer

<400>  16
aatctagacc tccggaaccc agaagctgtt ttgcggg                           37

## Description

The present invention involves an inhibitor in the field of immunology. It specifically involves a T-cell immune response inhibitor.

### Technical background

An organism's immune system is a complex regulation process throughout. Immunity regulation refers to the mutual functioning between the various cells in the immune system, between the immunity cells and the immunity molecules, and between the immune system and the other systems during the immune response process, all of which forms a mutually coordinating and mutually restraining network structure that maintains the immune response at the appropriate strength and thus ensures the stability of the organism's internal environment. After external pathogens invade, the immune system may, as determined by the characteristics of the pathogen, activate the immune response needed to resist and eliminate the pathogen. The immune response is further divided into the humoral immune response and the cellular immune response. The humoral immune response is a response produced by a specific antibody and the cellular immune response is an immune response that chiefly activates the T-cells. Vaccination is the principal method for improving an organism's immunity. At present there are many methods used to produce vaccines that resist infectious pathogens, for example, inactivated live vaccines, attenuated live vaccines, recombinant vaccines, subunit vaccines and DNA vaccines, among others. On a theoretical level, their basic functions are the same, namely, aided by the pathogen's antigen properties, vaccinated cells in the body identify and stimulate the immune response to achieve the goal of immunity in the individual so that the individual won't be infected by the pathogen. However, if an organism's immunity is too strong it may produce side effects, such as autoimmune disease. Therefore, when antigens invade from the outside, the organism may make use of a full complement of immunoregulatory mechanisms to equilibrate the immune response. Suppression of the immune response is one of the methods used to treat autoimmune disease in humans.

T-cell immuno-suppression is a crucial link in an organism's immunity function, for example, it limits the occurrence of autoimmune illness and down-regulates the immune response. T-cells may, when non-stimulating molecules are present, stimulate the APC cells through the T-cells or carry out the immuno-suppression function through the mutual interaction of the recently proved thymus source CD4⁺ CD25⁺ cells and new growth T-cells. In most autoimmune diseases, specific antigen receptors exist, for example, DNA-resistant antibodies found during clinical examination in the blood of systematic lupus erythematosus patients. These antibodies and antigens form immunity compounds that precipitate cyclical inflammation in the tissues. Furthermore, if the joint tissue of rheumatoid arthritis (RA) patients contains autoimmune response T-cells it may produce a response with certain unknown antigens. Not only can this type of T-cell identify a specific antigen through T-cell receptors (TCRs), it can also identify major histocompatibility (MHC) molecules.

Thus, autoimmune response antigen receptors identify early on the inflammation triggers that cause clinical systematic lupus erythematosus, rheumatoid arthritis and other serious autoimmune diseases.

Laboratory studies have confirmed that antigen receptors in certain autoimmune diseases, for example, NEB/NEW murine lupus erythematosus, experimental inoculation of myelin basic protein (MBP) and allergic encephalomyelitis (EAE) in murine and rat animal models.

In murine lupus erythematosus, use of anti-idiotypic antibody (anti-Ids) removal to produce B-cell autoimmune response achieves the therapeutic objectives. Some clinical cases indicate that anti-idiotypic antibodies can clearly slow illness; however, there are cases that show that anti-idiotypic antibodies worsen illness. Similarly, in the treatment of encephalomyelitis, immune TCR-derived peptides are used to resist autoimmune disease response TCRs. The results achieved remissive effects for some symptoms and some symptoms worsened.

Thus, when using immunization to treat certain autoimmune diseases, the patient's immune response directly affects clinical efficacy of the treatment. If the immunization causes the production of an antibody response and the formation of anti-Ids antibodies, these anti-Ids may possibly bring together B-cells or T-cells in the autoimmune response, triggering a regulatory lytic reaction in vitro to achieve remission of clinical symptoms; conversely, if the immune response causes the body to eliminate the anti-Ids, the immuno-reactant may then bind with B-cells or T-cells in the autoimmune response and it may also bind with their antigen receptors at the point of intersection, stimulating the immunity cells to produce even more autoimmune response antibodies (Abs) or T-cells, and causing clinical symptoms to worsen. The great majority of T-cells stimulated and activated by immunization may also trigger various types of helper T-cells, for example, the TH1 or TH2 response, and may cause the original potentially existing autoimmune disease symptoms to worsen, or cause symptoms to go into remission. Thus, more thorough research of immunological methodology is needed to effectively treat autoimmune disease.

Immuno-suppressants currently in general clinical use include chemical medications and antibodies. Of these, the chemical medications include Prograf (FK506), cyclosporin A (CsA), mycophenolate mofetil (MMF), azathioprine (Aza), prednisone (Pred) and methylprednisolone (MP). The antibodies are antilymphoblast globulin (ALG) and anti-CD4 monoclonal antibodies (OKT4). However, the preceding immuno-suppressants all have toxic side effects if used improperly. On the one hand, it may be that over-suppression of the organism's immune response causes many types of complications effects; on the other hand the body's own toxic side effects may cause exhaustion in organ functioning.

### Invention disclosures

The objective of the present invention is to supply an inhibitor that selectively inhibits the T-cell immune response.

The T-cell immune response inhibitors supplied in the present invention include targeted pathogen nucleic acid vaccines and the protein antigen expression of said nucleic acid vaccines; or it includes targeted pathogen nucleic acid vaccines and the active polypeptides of said nucleic acid vaccine's expression protein antigens; or it includes the inactivated pathogen and the targeted pathogen nucleic acid vaccines.

When the described T-cell immune response inhibitor includes individually packaged or mixed targeted pathogen nucleic acid vaccines and said nucleic acid vaccine expression protein antigen, the targeted pathogen nucleic acid vaccine and said nucleic acid vaccine expression protein antigen's physical proportion may be 2:1 to 10:1, optimally 5:1, in the described T-cell immune response inhibitor.

When the described T-cell immune response inhibitor includes individually packaged or mixed targeted pathogen nucleic acid vaccine and said nucleic acid vaccine expression protein antigen's active polypeptide, the targeted pathogen nucleic acid vaccine and said nucleic acid vaccine expression protein antigen's active polypeptide's physical proportion is 1:5 to 5:1 in the described T-cell immune response inhibitor.

When the described T-cell immune response inhibitor includes individually packaged or mixed inactivated pathogen and targeted pathogen nucleic acid vaccine, the inactivated pathogen and targeted pathogen nucleic acid vaccine's physical proportion is 1:2 to 1:10 in the described T-cell immune response inhibitor.

The described T-cell immune response inhibitor may also include an immunological adjuvant, for example, mineral oil (injection-use white camphor oil).

The described nucleic acid vaccine is a eukaryote cell expression carrier that contains protein antigen encoded genes.

In the described eukaryote cell expression carrier, the regulation and control protein antigen encoded gene expression promoters may be RSV (Rous sarcoma virus), CMV (cytomegalovirus) and SV40 viral promoters.

The described eukaryote cell expression carriers may be a plasmid expression carrier, a viral or bacteriophage expression carrier, an expression carrier composed of plasmid DNA and viral or bacteriophage DNA; an expression carrier composed of plasmid DNA and chromosomal DNA fragment and other expression carriers commonly used in the field of genetic engineering.

The described protein antigen-encoded gene's DNA may be double-stranded DNA artificially synthesized or extracted from microbes, eukaryotes and plant cells or tissues.

The protein in the described protein antigen is artificially synthesized or biologically produced protein.

The active polypeptides in the described protein antigen are artificially synthesized or biologically produced.

The described biological organisms may be produced using enhanced Escherichia coli or bacillocin or saccharomycete or other eukaryote cellular organisms under artificial culture conditions.

The described inactivated pathogens are noninfectious pathogens obtained through viruses, bacteria, parasites and allergenic substances isolated and produced from biological organisms after inactivation using commonly known methods.

The described inactivated pathogen may be directly mixed with nucleic acid vaccine or mixed with nucleic acid vaccine after emulsification with mineral oil (injection-use white camphor oil).

The described T-cell immune response inhibitor may be introduced into the organism muscularly, intracutaneously, subcutaneously, venously and through mucosal tissue by means of injection, spraying, oral administration, nose drops, eye drops, penetration, absorption, physical or chemical means; or it may be introduced into the organism through other physical mixture or package.

### Specifications for attached figures.

Figure 1 is 1% agarose gel electrophoresis of PCR expansion FMDV VP1 gene.
Figure 2 is enzyme splice assay electrophoresis conducted on the SuperY/VP1 recombinant expression carrier.
Figure 3 is the VP1 genetic expression product's SDS-PAGE spectrograph.
Figure 4 is the Western-blot test of VP1 protein expression.
Figure 5 measures changes in the properties of pcD-VP1 and 146S antigens after mixing.
Figure 6 shows the ELISA test results of antibody production after vaccinating mice with T-cell immune response inhibitors.
Figure 7a shows the influence of a T-cell immune response inhibitor formed of targeted pathogen nucleic acid vaccine and said nucleic acid vaccine's expression protein antigen on T-cell specificity expansion in vaccinated mice.
Figure 7b shows the influence of a T-cell immune response inhibitor formed of inactivated pathogen vaccine and targeted pathogen nucleic acid vaccine on T-cell specificity expansion in vaccinated mice.
Figure 8 shows the influence of a T-cell immune response inhibitor formed of inactivated pathogen vaccine and nucleic acid vaccine targeted at said pathogen on T-cell specificity expansion with common immunity at the same site or single immunity for different sites in vaccinated mice.
Figure 9 shows the influence of a pcD-S2 and recombinant hepatitis B surface antigen S protein T-cell immune response inhibitor on T-cell specificity expansion in vaccinated mice.
Figure 10 is a bar graph of the volume-effectiveness relationship for suppression of T-cell activity.
Figure 11 compares the influence of T-cell immune response inhibitors on interleukin levels in vaccinated mice.

### Preferred embodiments for the invention

Unless specified, the test methods mentioned in the following embodiments all refer to conventional methods. Where unspecified, the percent contents referenced are all mass percent contents.

### DNA formulation

A method that grinds animal tissue after it is brought to a low temperature, removes protein in a phenol chloroform solution and isolates the double-stranded DNA using ethyl alcohol.

Another method uses the CTAB method to extract DNA from plant tissue, removes protein in a phenol chloroform solution and has the double-stranded DNA undergo ethyl alcohol precipitation to separate out.

Another method extracts plasmid DNA from Escherichia coli, removes protein in a phenol chloroform solution and isolates the double-stranded DNA using ethyl alcohol precipitation.

Details for the preceding extraction methods and technologies may be referenced in Sambrook et al. Molecular Cloning (Cold Spring Harbor Laboratory Press, N.Y., 2nd edition, 1998) and Chaolong Li et al., editors, Experimental Technologies in Biochemistry and Molecular Biology (Zhejiang University Press).

### Protein and polypeptide formulation.

Proteins and polypeptides may be synthesized using standard automatic polypeptide synthesis instruments (for example, ABI, 433A, etc.) and the instrument manufacturer's usage methods; or it may be extracted from animal tissues and cells, plant tissues and cells or microorganisms in accordance with routine protein chemical methods. They may also be extracted from genetic engineering expression bacteria or cells. These polypeptide extraction methods are commonly known; for details refer to Doonan's Protein Purification Protocols (Humana Press, N.J., 1996).

### Pathogen formulation and inactivation

Pathogens are separated and produced from biological organisms such as viruses, bacteria, mycoplasma, parasites and allergic substances using commonly known inactivation methods and reagents, for example, formaldehyde or formalin, β-propiolactone, N-acetyl-vinyl-imide and divinyl-imide. After inactivation, noninfectious pathogens are obtained and put through separation and purification. Then preparation is complete.

### Embodiment 1. Bovine foot and mouth disease (FMDV) VP1 protein antigen preparation

### One. Bovine foot and mouth disease VP1 cDNA clones

Stomatic pathology tissue from cows infected with bovine foot and mouth disease virus and an RNA extraction reagent kit (purchased from the Shanghai Bioengineering Company) were used. In accordance with test kit instructions, the sulfocarbamidine one-step method was used to obtain total viral RNA. The specific procedures are as follows: crush and separate the pathology tissue cells, add 0.5mL sulfocarbamidine solution, 0.5mL phenol/ chloroform/ isopentanol (25:24:1) solution, at 4°C and 12,000rpm centrifuge for 5 minutes, transfer the supernatant to a new 1.5 microliter plastic centrifuge tube, add the remaining quantity of isopentanol, place at -20°C for 30 minutes, centrifuge at 12,000rpm for 10 minutes, remove the supernatant fluid, precipitate using 70% alcohol wash and once the precipitate dries dissolve in 30µL DEPC treated water. Denatured agarose gel electrophoresis test results indicate the viral RNA obtained.

Under the following response conditions, the first strand of cDNA is synthesized: 2 µg bovine foot and mouth disease viral RNA, 50mmol/L Tris-HCl (pH8.3), 75mmol/L KCl, 10mmol/L DTT, 3mmol/L MgCl₂, 500µ mol/L dNTPs, 100µg of six random polymer primers, 500 units of MMLV reverse transcriptase, to a total volume of 20µL and maintain at a temperature of 37°C for one hour. Using the first strand of cDNA product as a template, under the guidance of primer 1:5' - AAG AATTCGGAGGTACCACCTCTGCGGGTGAG-3' and primer 2: 5' - AATCTAGACCTCCGGAACCCAGAAGCTGTTTTGCGGG-3' (at primer 1 and primer 2, introduce the EcoRI identifier site and XbaI identifier site, respectively) perform PCR expansion of bovine foot and mouth disease virus VPI cDNA. Response system: 5 µL first strand cDNA product, primer 1 and primer 2 are 10pmol, 500mM KCl, 100mM Tris-HCl (pH 8.4), 1.5mM MgCl₂, 100 µg/mL BSA, 1mM dNTPs, 2.5U Taq DNA polymerase, to a total volume of 50 µL. Response conditions are: 94°C denaturation for 30 seconds, 54°C renaturation for 30 seconds, and extend at 72°C for one minute, for a total of 30 cycles. The PCR expansion FMDV VP1 gene's 1% agarose gel electrophoresis test results are as shown in Table 1 (lane M is the DNA marker; lane 1 is the PCR product). In the figure the tip of the arrow indicates the target band site, the indicated target fragment's size is 639bp, consistent with the size of the VP1 gene fragment. Low fusion point gel is used to collect the expansion fragment.

### Two. Expression carrier Super Y/VP1 structure and assay

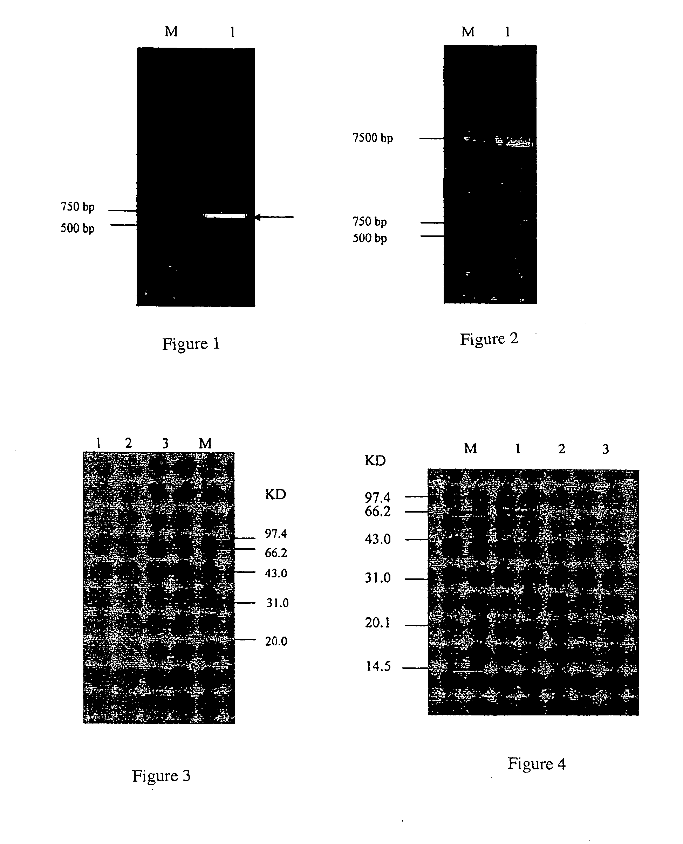

Using restriction endonuclease EcoRI and XbaI digestion procedures to obtain bovine foot and mouth disease VP1 cDNA fragments perform electrophoresis. After collection, insert the VP1 gene fragment clone into plasmid SuperY (to plasmid at pGAPZa SphI and HpaI sites purchased from U.S. Invitrogen Company add kanamycin resistance gene {Kan^{r}} to obtain SuperY) between the EcoRI and XbaI digestion sites, then use EcoRI and XbaI restriction endonuclease to conduct the digestion assay on the recombinant carrier, use the digestion product to perform 1% agarose gel electrophoresis. The test results are shown in Figure 2 (lane M is the DNA marker, lane 1 is enzyme splice product), wherein the size of the small fragment is 639bp, consistent with the size of the VP1 gene fragment, indicating that VP1 is already corrected on the clone at SuperY, assign the name SuperY/VP1 to said recombinant carrier, then convert the SuperY/VP1 to Escherichia coli Top 10 F' competent cells, filter to select the assay's positive clone and conduct sequential analysis on the positive clone. The results indicate that the expansion product's nucleotide sequence is consistent with the VP1 gene and has been successfully cloned at the SuperY plasmid.

### Three. Testing of VP1 gene in yeast expression and its expression product

Take the recombinant expression carrier SuperY/VP1 constructed in procedure two and use the electroshock method to convert it to yeast SMD1168. Filter out the assay's positive clone, select out a single bacterial colony, after agitating the flask, culture at 30°C for 48-96
hours (at the same time designate it yeast SMD1168 and convert the yeast SMD1168 with
SuperY into the control). After supernatant denaturation, conduct SDS-PAGE electrophoresis. After Coommassie brilliant blue color G250 staining, use the gel imaging system to produce the photographs. The results are shown in Figure 3 (lane 1: yeast SMD1168 supernatant; lane 2: converted SuperY yeast SMD1168 expression supernatant; lane 3: converted Super Y yeast SMD1168 expression supernatant; lane M: low molecular weight protein standard). From lane 3 we learn that there are two types of VP1 expression products of molecular weights 66kD and 43kD, indicating that the VP1 gene in the SuperY/VP1 achieves expression in yeast cells. Use said recombinant expression carrier SuperY/VP1's expression product to conduct Western blotting analysis. The specific methodology is: After obtaining the denatured expression protein, use SDS-PAGE to separate the protein, then electronically transfer it to NC film and use 5% fat-free milk as a sealant. Next, use anti-bovine foot and mouth disease virus hyperimmune serum (purchased from the Xinjiang Construction Unit General Veterinary Station) and anti-sheep cow IgG-HRP enzyme label antibody (purchased from the U.S. Sigma Company). Incubate and then develop in DAB/H₂O₂. The results are shown in Figure 4 (lane M: low molecular weight protein standard; lane 1: converted SuperY/VP1 yeast SMD1168 expression supernatant; lane 3: yeast SMD1168 supernatant; lane 4: converted SuperY yeast SMD1168 expression supernatant). At lane 1 near 66kD and 43kD, specific color bands appear, and in lanes 2 and 3 no bands appear, indicating that the expression protein is able to produce a specific response band with the anti-FMDV serum response and the expression protein product possesses FMDV immunogeneity. After the expression supernatant is desalinated and purified, it is stored at -20°C. It may be used as bovine foot and mouth disease VP1 protein vaccine in the following embodiments.

### Embodiment 2 measures the properties of the targeted pathogen's nucleic acid vaccine and said nucleic acid vaccine's expression protein antibody compounds

Using restriction endonuclease EcoRI and XbaI to perform digestion in procedure 1 of Embodiment 1, obtain foot and mouth disease VP1 cDNA fragments. Collect the VP1 gene, use the eukaryotic expression plasmid pcDNA3 to perform digestion exactly as EcoRI and XbaI. Use T₄DNA ligase to connect the VP1 gene fragment at pcDNA3 (purchased from U.S. Invitrogen Company). Convert to Escherichia coli DH5a competent cells. On the plate, filter to select ampicillin (50µg/mL) resistant colonies, obtain plasmid, perform digestion filter assay for the correct clone, and obtain recombinant plasmid pcD-VP1 containing the VP1 gene.

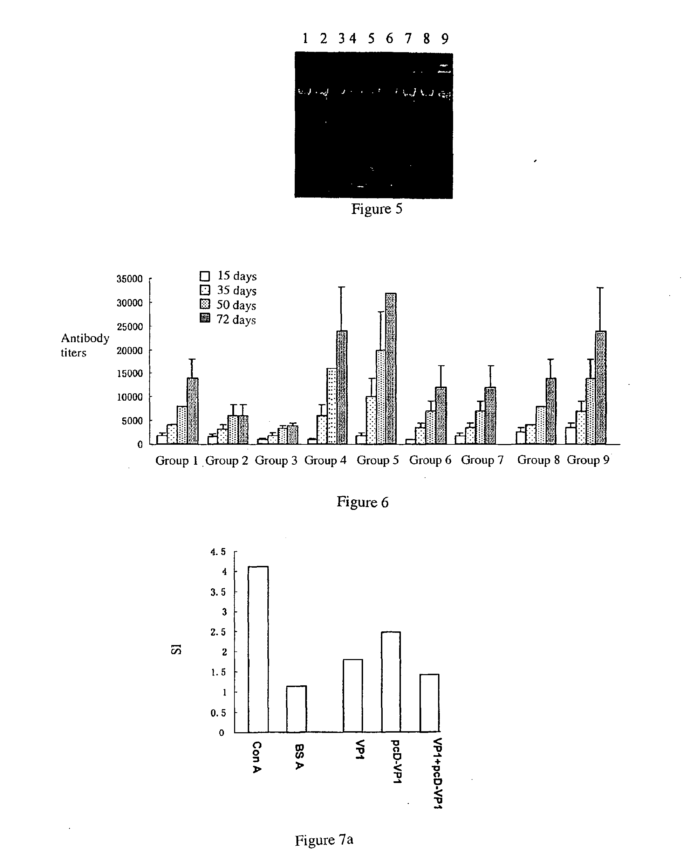

To prove that the targeted pathogen's nucleic acid vaccine and said nucleic acid vaccine's expression protein antigen do not change in a tangible way after mixing, take the remaining quantity of pcD-VP1 and 146S antigen (remove the mineral oil from the bovine foot and mouth disease inactivated O-type vaccine {purchased from the Lanzhou Veterinary Medicine Research Institute} to obtain the 146S antigen). After mixing, place at 37°C and incubate for 24 hours, perform 1% agarose gel electrophoresis and compare the changes. The results are shown in Figure 5, which indicates that there are no changes before and after the pcD-VP1 and 146S antigen are mixed together. In the figure, lanes 1 and 2 are pcD-VP1 levels prior to mixing; 3 and 4 shows the details of the blended pcD-VP1 and 146s samples after electrophoresis; lanes 5 and 6 show the details of the blended pcD-VP1 and 146s sample after 24 hours of incubation at 37°C; lanes 7 and 8 show the details of the blended pcD-VP1 and 146s sample after the addition of 10 units of DNA enzyme I (Sigma Company) and 24 hours of incubation at 37°C. Lane 9 is a DNA Marker.

### Embodiment 3. ELISA detection of antibodies produced after vaccinating mice with T-cell immune response inhibitors

In order to verify the impact of a T-cell immune response inhibitor formed of a nucleic acid vaccine for a targeted pathogen and said nucleic acid vaccine's expression protein antigen and a T-cell immune response inhibitor formed of an inactivated pathogen and a nucleic acid vaccine for said targeted pathogen on immunity levels in the immune systems of vaccinated mice, the following animal tests were performed.

Divide 54 BALB/c (H-2^{d}) female mice 6-8 weeks old into 9 groups, 6 animals per group. The first group receives an intramuscular injection of 100 microliters of 20 micrograms of bovine foot and mouth disease inactivated O-type vaccine (purchased from the Lanzhou Veterinary Medicine Research Institute); at 14 days a single booster is administered in the same dosage. The second group receives an intramuscular injection of 100 microliters of a 20- microgram VP1 protein 0.9% NaCl aqueous solution; at 14 days a single booster is administered in the same dosage. The third group receives an intramuscular injection of 100 microliters of a 100-microgram pcD-VP1 protein 0.9% NaCl aqueous solution; at 14 days a single booster is administered in the same dosage. The fourth group receives an intramuscular injection of 100 microliters of a 100-microgram pcD-VP1 protein 0.9% NaCl aqueous solution; at 14 days after the first vaccination, an intramuscular injection of 100 microliters of a 20-microgram bovine foot and mouth disease inactivated O-type vaccine is administered. The fifth group receives an intramuscular injection of 100 microliters of 20-microgram bovine foot and mouth disease inactivated O-type vaccine; at 14 days after the first vaccination, an injection of 100 microliters of a 100-microgram pcD-VP1 0.9% NaCl aqueous solution is administered. The sixth group receives an intramuscular injection of 100 microliters of a 100-microgram pcD-VP1 0.9% NaCl aqueous solution; at 14 days after the first vaccination, a single injection of 100 microliters of a 20-microgram VP1 protein 0.9% NaCl aqueous solution is administered. The seventh group receives an intramuscular injection of 100 microliters of a 20-microgram VP1 protein 0.9% NaCl aqueous solution; at 14 days after the first vaccination, a single injection of 100 microliters of a 100-microgram pcD-VP1 0.9% NaCl aqueous solution is administered. The eighth group receives an intramuscular injection of 100 microliters of 0.9% NaCl aqueous solution containing 100 micrograms of pcD-VP1 and 20 micrograms of VP1 protein; at 14 days a single booster injection in the same dosage is administered. The ninth group receives an intramuscular injection of 100 microliters of a mixture solution containing 100 micrograms of pcD-VP1 and 20 micrograms of bovine foot and mouth disease inactivated O-type vaccine; at 14 days a single booster in the same dosage is administered and then at 15, 35, 50 and 72 days sera is obtained to perform antibody titers using the ELISA method. The test methodology is: Use a 96-well enzyme label plate with 8ug/ml antigen pockets, store at 4°C overnight. Seal 3% calf sera at 37°C for one hour; use PBST (0.05% Tween20 dissolved in PBS) to wash three times, five minutes each time. Add no less than series dilution of immunized animal (murine) serum. Use non-immunized murine sera as the control and incubate at 37°C for two hours. After washing the plate with PBST three times, add to each well 100µL horseradish peroxide enzyme-labeled sheep anti-mouse IgG (Sigma, St. Louis). Remove after incubating at 37°C for one hour. Wash with PBST three times, five minutes each time. Wash with PBST three times then add 100µL substrate TMB fluid. The visible response occurs after 30 minutes at room temperature. 2M sulfuric acid stops the response. Use the enzyme label instrument to measure the OD_{480, 620} optical density signal. When the OD values of the experimental well reach double the OD values of the control wells, they are considered positive. The results in Figure 6 indicate that after mice are vaccinated with the T-cell immune response inhibitor formed of nucleic acid vaccine pcD-VP1 and pcD-VP1 expression protein antigen VP1 and the T-cell immune response inhibitor formed of bovine foot and mouth disease inactivated O-type vaccine and pcD-VP1, there are no clear changes to specific antibody levels in comparison with other groups. The explanation is that after the animal is vaccinated with T-cell immune response inhibitor formed of the nucleic acid vaccine for the targeted pathogen and said nucleic acid vaccine's expression protein antigen and the T-cell immune response inhibitor formed of the inactivated pathogen and the nucleic acid vaccine for said targeted pathogen, there are no changes to the specific antibody levels stimulated. In Figure 6, ELISA serology results are shown for each group from left to right at 15, 35, 50 and 72 days after the second vaccination. (The X-axis in Figure 6 indicates the immunized group.)

### Embodiment 4. The impact of a T-cell immunity response inhibitor formed of a nucleic acid vaccine for a targeted pathogen and said nucleic acid vaccine's expression protein antigen on the specific T-cell expansion of immunized mice

Divide 30 BALB/c (H-2d) female mice 6-8 weeks old into three groups. The first group receives an intramuscular injection of 100 microliters of a 20-microgram VP1 protein 0.9% NaCl aqueous solution. The second group receives an intramuscular injection of 100 microliters of a 100-microgram nucleic acid vaccine pcD-VP1 0.9% NaCl aqueous solution. The third group receives an intramuscular injection of 100 microliters of 0.9% NaCl aqueous solution containing 100 micrograms of nucleic acid pcD-VP1 and 20 micrograms of VP1 protein; at 14 days after the first vaccination, a single booster is administered in the same dosage and then 14 days after the second vaccination spleen T cells were obtained to measure T-cell expansion activity. The specific methodology is: Under antiseptic conditions, the spleen is prepared as a single cell suspension. Use hemolytic solution to remove red blood cells, then wash three times using PBS fluid, centrifuge and take the cell count, adjust cell concentrations to 1 × 10⁶ parts/ml, divide each cell suspension into four parts and add to a 96-well culture plate. To one part add 100µl Con A (mitogen) to a final concentration of 5µg/ml. To one part add the corresponding specific antigen (VP1) to serve as stimulant for a final concentration of 2µg/ml. To one part add no stimulant. To one part add 100µl BSA to a final concentration of 2µg/ml to serve as an unrelated antigen. Then 24 hours later, add 100µl MTT to each well for a final concentration of 5mg/ml.

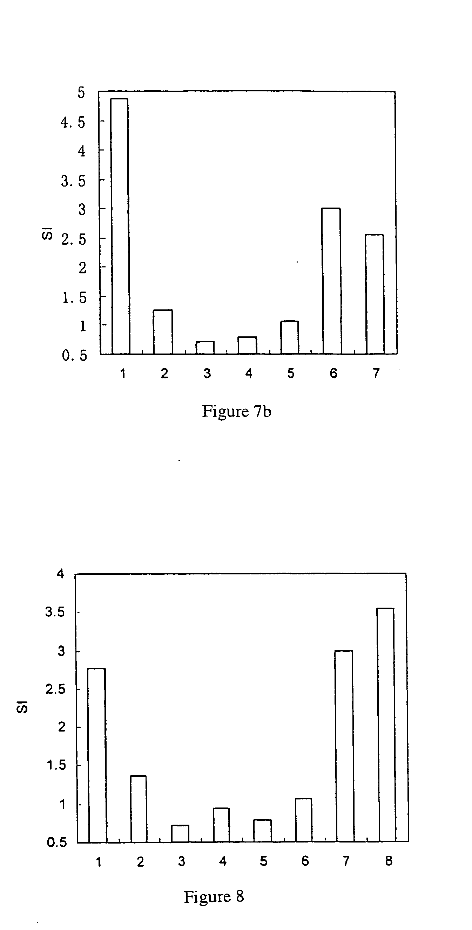

Next, 48 hours later, add 100µl SDS-DMSO (dissolve 20% SDS in 50% DMSO, pH2.0) to each well and dissolve completely. After 4h incubation, use the enzyme labeler to read the OD value at 570nm and calculate the stimulation index SI (SI = experimental stimulation count ÷ non-stimulation count). The results in Figure 7a indicate that the T-cell expansion activity of an animal immunized with a T-cell immune response inhibitor containing nucleic acid vaccine pcD-VP1 and VP1 is clearly lower than that of the nucleic acid vaccine group and the VP1 group. The explanation is that the nucleic acid vaccine pcD-VP1 and VP1 T-cell immune response inhibitor may reduce the specificity of T-cell immunity levels. In Figure 7b, Con A indicates the positive control; BSA is the negative control; VP1 is the first group; pcD-VP1 is the second group; and VP1 + pcD-VP1 is the third group.

### Embodiment 5 is the impact of a T-cell immune response inhibitor formed of inactivated pathogen vaccine and the nucleic acid vaccine for the targeted pathogen on T-cell specificity expansion in immunized mice.

Divide 50 BALB/c (H-2d) female mice 6-8 weeks old into five groups. The first group receives an intramuscular injection of 100 microliters of 0.9% NaCl aqueous solution containing 100 micrograms of nucleic acid vaccine pcD-VP1 and 20 micrograms of 146S antigen (the oil is removed from bovine foot and mouth disease inactivated O-type vaccine, purchased from the Lanzhou Veterinary Medicine Research Institute). The second group receives an intramuscular injection of 100 microliters of a mixture solution containing 100 micrograms of nucleic acid vaccine pcD-VP1 and 20 micrograms of bovine foot and mouth disease inactivated O-type vaccine (purchased from the Lanzhou Veterinary Medicine Research Institute). The third group receives an intramuscular injection of 100 microliters containing 20 micrograms of bovine foot and mouth disease inactivated O-type vaccine. The fourth group receives an intramuscular injection of 100 microliters of 0.9% NaCl aqueous solution containing 100 micrograms of nucleic acid vaccine pcD-VP1. The fifth group receives an intramuscular injection of 100 microliters of a mixture solution containing 100 micrograms of nucleic acid vaccine pcD-VP1 and 20 micrograms of porcine reproductive and respiratory system virus (PRRSV) inactivated vaccine (purchased from the Harbin Veterinary Medicine Research Institute). At 14 days after the first immunization, a single booster in the same dosage is administered; and at 14 days after the second vaccination, spleen T cells are obtained to measure their T-cell expansion activity. The specific methodology is: Under antiseptic conditions, prepare the spleen as a single cell suspension. Use hemolytic solution to remove red blood cells, then wash three times using PBS fluid, centrifuge and take cell count, adjust cell concentrations to 1 × 10⁶ parts/ml, divide each cell suspension into four parts and add to a 96-well culture plate. To one part add 100µl Con A (mitogen) to a final concentration of 5µg/ml. To one part add the corresponding specific antigen (146S antigen) to serve as stimulant for a final concentration of 2µg/ml. To one part add no stimulant and to one part add 100µl BSA to a final concentration of 2µg/ml to serve as an unrelated antigen. Then 24 hours later, add 100µl MTT to each well for a final concentration of 5mg/ml. Then 48 hours later, add 100µl SDS-DMSO (dissolve 20% SDS in 50% DMSO, pH2.0) to each well and dissolve completely. After 4h incubation, use the enzyme labeler to read the OD value at 570nm and calculate the stimulation index SI (SI = experimental stimulation count ÷ non-stimulation count). Figure 7b shows the results for animals immunized with a T-cell immunity response inhibitor containing nucleic acid vaccine pcD-VP1 and 20 micrograms bovine foot and mouth disease O- type inactivated vaccine and a T-cell immunity response inhibitor containing pcD-VP1 nucleic acid vaccine and 146S antigen. T-cell expansion activity is clearly lower than that of the nucleic acid group or the bovine foot and mouth disease inactivated O-type vaccine group and the nucleic acid vaccine pcD-VP1 and inactivated porcine reproductive and respiratory system vaccine group. The explanation is that its suppressed T-cell expansion activity is antigen-specific. In Figure 7b, 1. is the Con A positive control; 2. is the BSA non-specific antigen group; 3. is the pcD-VP1 nucleic acid vaccine and 146S antigen vaccine shared immunity group; 4. is the pcD-VP1 nucleic acid vaccine and bovine foot and mouth disease inactivated O-type vaccine shared immunity group; 5. is bovine foot and mouth disease inactivated O-type vaccine; 6. is nucleic acid vaccine pcD-VP1 immunity group; 7. is pcD-VP1 nucleic acid vaccine and inactivated porcine reproductive and respiratory system vaccine shared immunity group.

### Embodiment 6. The influence of a T-cell immune response inhibitor formed of inactivated pathogen vaccine and nucleic acid vaccine targeting said pathogen on T-cell specificity expansion with common immunity at the same site or single immunity for different sites in immunized mice.

The impact of the T-cell immune response inhibitor formed of the inactivated pathogen vaccine and the targeted pathogen nucleic acid vaccine on T-cell specificity expansion in immunized mice.

Divide 60 BALB/c (H-2d) female mice 6-8 weeks old into six groups. The first group receives an intramuscular injection of 100 microliters of a compound formed of 100 micrograms of nucleic acid vaccine pcD-VP1 and 20 micrograms of 146S antigen (the oil is removed from bovine foot and mouth disease inactivated O-type vaccine antigen) in a 0.9% NaCl aqueous solution; the second group receives an intramuscular injection in the left foot of 50 microliters of a 20-microgram bovine foot and mouth disease inactivated O-type vaccine in a 0.9% NaCl aqueous solution and an intramuscular injection in the right foot of 50 microliters of 100 micrograms of nucleic acid vaccine pcD-VP1 in a 0.9% NaCl aqueous solution; the third group receives an intramuscular injection of 100 microliters of a 100-microgram nucleic acid vaccine pcD-VP1 and 20-microgram bovine foot and mouth disease inactivated O-type vaccine 0.9% NaCl aqueous solution; the fourth group receives an intramuscular injection of 100 microliters of a 20-microgram bovine foot and mouth disease inactivated O-type vaccine; the fifth group receives an intramuscular injection of 100 microliters of a 100-microgram nucleic acid vaccine pcD-VP1 0.9% NaCl aqueous solution; the sixth group receives an intramuscular injection of 100 microliters of a 0.9% NaCl aqueous solution containing 100 micrograms of nucleic acid vaccine pcD-VP1 and 20 micrograms of porcine reproductive and respiratory system vaccine (PRRSV) inactivated virus vaccine (purchased from the Harbin Veterinary Medicine Research Institute). At 14 days after the first immunization, a single booster in the same dosage is administered; and at 14 days after the second dose, spleen T cells are obtained to measure T-cell expansion activity. The specific methodology is the same as that in Embodiment 5. The results are shown in Figure 8 and indicate that whether or not the T-cell immune response inhibitor contains an oil adjuvant, animals immunized with a T-cell immune response inhibitor containing nucleic acid vaccine pcD-VP1 and foot and mouth disease 146S antigen. Its T-cell expansion activity is clearly lower than that of the nucleic acid group or that of the bovine foot and mouth disease inactivated O-type vaccine and the nucleic acid vaccine pcD-VP1 and inactivated porcine reproductive and respiratory system vaccine group. The explanation is that suppression of this T-cell expansion activity is antigen specific and it proves that whether the nucleic acid pcD-VP1 and foot and mouth disease 146S antigen have shared immunity at the same site or separate immunity at different sites, it can suppress T-cell activity. In Figure 8, 1. is the Con A positive control; 2. is the BSA non-specific antigen group; 3. is the pcD-VP1 nucleic acid vaccine and 146S antigen shared immunity group; 4. is the left foot intramuscular injection 146S antigen and the right foot intramuscular injection pcD-VP1 nucleic acid vaccine group; 5. is pcD-VP1 nucleic acid vaccine and bovine foot and mouth disease inactivated O-type vaccine shared immunity group; 6. is bovine foot and mouth disease inactivated O-type vaccine; 7. is the nucleic acid vaccine pcD-VP1 immunity group; 8. is the pcD-VP1 nucleic acid vaccine and inactivated porcine reproductive and respiratory system vaccine shared immunity group.

### Embodiment 7. The impact of a T-cell immune response inhibitor formed of the pathogenic antigen and the nucleic acid vaccine for said targeted pathogenic antigen on T-cell specificity expansion in immunized mice

Using the total length of the HBV gene group in pADR plasmid (Gan RB, Cu MJ, Li ZP, et al. The complete nucleotide sequence of the cloned DNA of hepatitis B virus subtype adr in pADR-1. Sci Sin (B), 1987, 30 (5): 507-521) as the protocol, at primer 1: 5' -CGGATCCATTAAGCCATGCAGTGGAACTCC-3'; and primer 2: 5' - GTCCTTGGGTATACATTTGAACCCCGGATCCA - 3', (at primer 1 and primer 2, insert the Bam HI identifier site, at the same time at primer 1 introduce initiator site ATG, at primer 2 introduce termination site TGA) guided PCR expansion HBV S2 antigen DNA fragment. The response system: 5µL pADR plasmid (10ng), primer 1 and primer 2 are each 10pmol, 500mM KCl, 100mM Tris-HCl (pH8.4), 1.5mM MgCl₂, 100 µg/mL BSA, 1mM dNTPs, 2.5U Taq DNA polymerase and total volume is 50µL. The response conditions are: 94°C denaturation for 30 seconds, 54°C renaturation for 30 seconds, 72°C extension for 1 minute, for a total of 30 cycles. For the PCR expansion's DNA fragment product, use the restriction endonuclease BamHI for digestion, collect HBV S2 antigen DNA fragments, use eukaryotic expression plasmid pcDNA3 for the same BamHI digestion, use T₄ DNA ligase to attach the S2 gene fragment to pcDNA3 (purchased from Invitrogen Company), convert to Escherichia coli DH5 α competent cells, on the plate, filter to select ampicillin (50 g/mL) resistant colonies, obtain plasmid, perform digestion filter assay to correct the clone and obtain recombinant plasmid pcD-S2 with S2 gene.

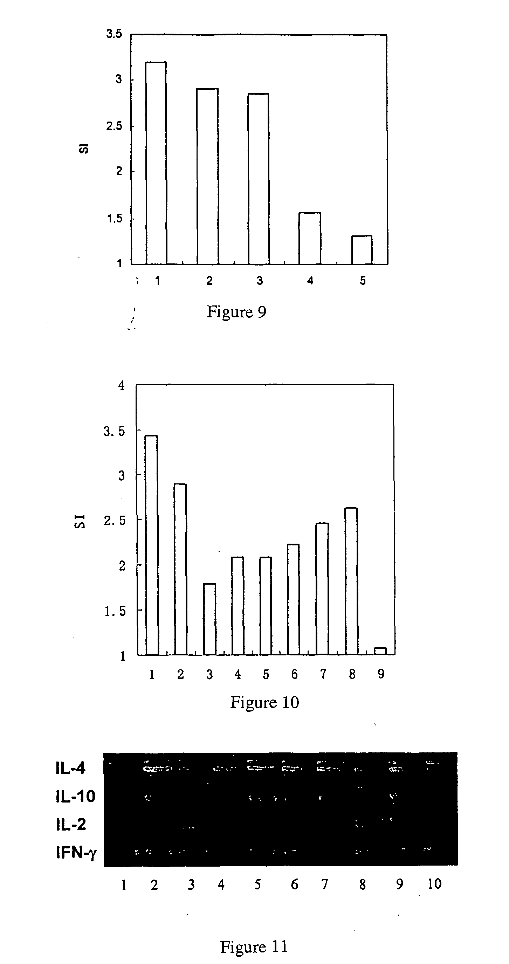

Divide 30 BALB/c (H-2d) female mice 6-8 weeks old into three groups. The first group receives an intramuscular injection of 100 microliters of a 0.9% NaCl aqueous solution containing 100 micrograms of recombinant hepatitis B surface antigen S gene nucleic acid vaccine pcD-S2; the second group receives an intramuscular injection of 100 microliters of a 0.9% NaCl aqueous solution containing 20 micrograms of recombinant hepatitis B surface antigen S protein (purchased from the Beijing Tiantan Biological Products Manufacturer) vaccine; the third group receives an intramuscular injection of 100 microliters of a 0.9% NaCl aqueous solution containing 100 micrograms of nucleic acid vaccine pcD-S2 and 20 micrograms of recombinant hepatitis B surface antigen S protein vaccine. At 14 days after the first immunization, a single booster in the same amount is administered; and at 14 days after the second dose, spleen T cells are obtained to test for T-cell expansion activity. The specific methodology ― except that the stimulant is recombinant hepatitis B surface antigen S protein - is identical to that in Embodiment 5. The results are shown in Figure 9 and indicate that the use of nucleic acid vaccine pcD-S2 and 20 micrograms of recombinant hepatitis B surface antigen S protein vaccine T-cell immune response inhibitor to immunize animals had a clearly lower effect on T-cell expansion activity than that of the nucleic acid group and the protein vaccine group. In Figure 9, 1. is the Con A positive control; 2. is the nucleic acid vaccine pcD-S2 immunity group; 3. is the recombinant hepatitis B surface antigen S protein vaccine immunity group; 4. is the nucleic acid vaccine pcD-S2 and recombinant hepatitis B surface antigen S protein vaccine immunity group; 5. is the BSA non-specific antigen group.

### Embodiment 8. The volume-effectiveness relationship in suppression of T-cell activity.

Divide 70 BALB/c (H-2d) female mice 6-8 weeks old into seven groups. The first group receives an intramuscular injection of 100 microliters of a 0.9% NaCl aqueous solution containing 100 micrograms of foot and mouth disease VP1 gene nucleic acid vaccine pcD-VP1; the second group receives an intramuscular injection of 100 microliters of a 0.9% NaCl aqueous solution containing 100 micrograms of nucleic acid vaccine pcD-VP1 and 20 micrograms of bovine foot and mouth disease inactivated virus vaccine (purchased from the Lanzhou Veterinary Medicine Research Institute, it contains 50% injection-use white camphor oil); the third group receives an intramuscular injection of 100 microliters of 0.9% NaCl aqueous solution containing 100 micrograms of nucleic acid vaccine pcD-VP1 and 20 micrograms of foot and mouth disease VP1 protein vaccine; the fourth group receives an intramuscular injection of 100 microliters of a 0.9% NaCl aqueous solution containing 100 micrograms of nucleic acid vaccine pcD-VP1 and 200 micrograms of foot and mouth disease VP1 protein RGD peptide (the sequence is: NH2-LRGDLQVLAQKVARTL-COOH) vaccine; the fifth group receives an intramuscular injection of 100 microliters of a 0.9% NaCl aqueous solution containing 100 micrograms of nucleic acid vaccine pcD-VP1 and 50 micrograms of foot and mouth disease VP1 protein RGD peptide vaccine; the sixth group receives an intramuscular injection of 100 microliters of a 0.9% NaCl aqueous solution containing 100 micrograms of nucleic acid vaccine pcD-VP1 and 12.5 micrograms of foot and mouth disease VP1 protein RGD peptide; the seventh group receives an intramuscular injection of 100 microliters of a 0.9% NaCl aqueous solution containing 100 micrograms of nucleic acid vaccine pcD-VP1 and 20 micrograms of porcine reproductive and respiratory system virus E2 antigen peptide vaccine (the sequence is: NH2-CTAVSPTTLRT-COOH). At 14 days after the first immunization, a single booster in the same dosage is administered; and at 14 days after the second immunization, spleen T cells are obtained to measure T-cell expansion activity. The specific methodology - except that the stimulant is VP1 protein or swine flu E2 peptide (seventh group) - is the same as that in Embodiment 5. The results are shown in Figure 10 and indicate that nucleic acid vaccine pcD-VP1 and recombinant VP1 protein vaccine share immunity in animals. Its T-cell expansion activity is clearly lower than that of the nucleic acid vaccine single immunity in the second group; at the same time it also indicates that nucleic acid vaccine pcD-VP1 and VP1 protein RGD peptide vaccine forms T-cell immune response inhibitors at different concentrations to co-immunize animals. Its T-cell expansion activity is clearly lower than that of the nucleic acid vaccine single immunity group and presented a volume-effectiveness relationship, that is, the higher the RGD peptide concentration, the clearer the T-cell expansion activity suppression. In Figure 10, 1. is the Con A positive control; 2. is the nucleic acid vaccine pcD-VP1 immunity group; 3. is the pcD-VP1 and foot and mouth disease inactivated virus vaccine immunity group; 4. is the pcD-VP1 and foot and mouth disease VP1 protein vaccine immunity group; 5. is the pcD-VP1 and 200-microgram foot and mouth disease VP1 protein RGD peptide vaccine compound immunity group; 6. is the pcD-VP1 and 50-microgram RGD peptide vaccine compound immunity group; 7. is the pcD-VP1 and 12.5-microgram RGD peptide vaccine compound immunity group; 8. is the pcD-VP1 and 20-microgram swine flu E2 antigen peptide vaccine compound; 9. is the BSA non-specific antigen group.

### Embodiment 9. Detecting cell factor levels

Divide 60 BALB/c (H-2^{d}) female mice 6-8 weeks old into 10 groups of six each. The first group receives two intramuscular injections of 100 microliters of a 0.9% NaCl aqueous solution containing 100 micrograms of pcD-VP1, with a 14-day interval between the two injections. The second group receives two intramuscular injections of 100 microliters of a 20-microgram bovine foot and mouth disease inactivated O-type vaccine (purchased from the Lanzhou Veterinary Medicine Research Institute), with a 14-day interval between the two injections. The third group receives an intramuscular injection of 100 microliters of a 0.9% NaCl aqueous solution containing 100 micrograms of pcD-VP1, and 14 days later a second injection containing 20 micrograms of bovine foot and mouth disease inactivated O-type vaccine. The fourth group receives an intramuscular injection of 100 microliters of a 20-microgram bovine foot and mouth disease inactivated O-type vaccine, and 14 days later 100 microliters of a 0.9% NaCl aqueous solution containing 100 micrograms of pcD-VP1. The fifth group receives two intramuscular injections of 100 microliters of a mixture solution containing 100 micrograms of pcD-VP1 and 20 micrograms of bovine foot and mouth disease inactivated O-type vaccine, with a 14-day interval between the two injections. The sixth group receives two intramuscular injections of 100 microliters of a 0.9% NaCl aqueous solution containing 20 micrograms of VP1 protein, with a 14-day interval between the two injections. The seventh group receives a single intramuscular injection of 100 microliters of a 0.9% NaCl aqueous solution containing 20 micrograms of VP1 protein and after 14 days a second injection of a 0.9% NaCl aqueous solution containing 100 micrograms of pcD-VP1. The eighth group receives a first intramuscular injection of 100 microliters of a 0.9% NaCl aqueous solution containing 100 micrograms of pcD-VP1, and 14 days later a second intramuscular injection of 100 microliters of a 0.9% NaCl aqueous solution containing 20 micrograms of VP1. The ninth group receives two intramuscular injections of 100 microliters of a 0.9% NaCl solution containing 100 micrograms of pcD-V1 and 200 micrograms of VP1, with a 14-day interval between the two injections. The tenth group receives an intramuscular injection of 100 microliters of a 0.9% NaCl aqueous solution as a control.

Use of polycompetitor PCR to conduct testing of cell factor mRNA levels is key to introducing an internal standard protocol pQRS that contains IL-2, IL-4, IL-10, IFN-γ, HRPT and other genes in a partial sequence (add a section of 50-60bp nucleotides to pQRS plasmid in each gene, to make its gene-to-wild model IL-2, IL-4, IL-10, IFN-γ and HRPT other genes greater. After using the same kind of primer expansion, based on size we can determine the difference between the internal standard protocol and the wild protocol. At the same time, because of the competitive relationship, we can determine the volume relationship between the wild protocol and the internal standard protocol. For the specific preparation method, refer to the Journal of Immunological Methods, 1993, 165:37, "Constructing polycompetitor cDNAs for quantitative PCR." Thus, by using pQRS as the internal standard protocol it is possible to detect the amount of the corresponding cell factor in the immunized animal (Jin Huali et al. in an article appearing on page 2925 of issue 22 of the journal Vaccine in 2004: Effect of Chemical Adjuvants on DNA Vaccination).

After being vaccinated, the spleen is removed through the necks of the mice and total RNA (TRIZOL, Dingguo Biological Company) obtained. Reverse transcription is cDNA, and reverse transcription is performed in accordance with the RNA RT-PCR operating handbook from the Dalianbao Company to obtain 1µg of purified total RNA. It is placed in a 250µL centrifuge tube and then the corresponding reagent is added: 4µl MgCl₂, 2µl 10× buffer solution, 8.5µl DEPC water, 2µl dNTP mixture, 0.5µl RNase inhibitor, 0.5µl M-MLV reverse transcriptase (Promage Company), 0.5µL Oligo (dT)₁₂ primer; the response conditions are 42°C for 30min, 99°C for 5min and 5°C for 5min. Use Kan gene family hypoxanthine phosphoribosyltransferase (HPRT) as the internal source expression standard, adjust to be consistent with the cDNA concentrations of the various groups, then add 2µl cDNA to a 100ng pQRS tube to conduct PCR expansion. Because of the pQRS competition, the expansion quantities of the four cell factors below, IL-2 gene, IFN-γ gene, IL-4 gene and IL-10 gene, will clearly correlate to pQRS expansion quantities and have different reactions at different concentrations of electrophoresis gel. The primer required for the response and the PCR response conditions are shown in Table 1.

**Table 1. HPRT, IL-2, IFN-γ, IL-4 and IL-10 Primer Sequence and PCR Response.**

| Target gene | Primer | Response conditions |
|---|---|---|
| HPRT | 5' GTTGGATACAGGCCAGACTTTGTTG 3' | 94°C 30 sec, 60°C 30 sec and 72°C 40 sec |
| | GAGGGTAGGCTGGCCTATGGCT | |
| IL-2 | 5'TCCACTTCAAGCTCTACAG 3' | 94°C 30 sec, 55°C 30 sec and 72°C 40 sec |
| | GAGTCAAATCCAGAACATGCC | |
| IFN-γ | 5' CATTGAAAGCCTAGAAAGTCTG 3' | 94°C 30 sec, 58°C 30 sec and 72°C 40 sec |
| | CTCATGGAATGCATCCTTTTTCG | |
| IL-4 | 5' GAAAGAGACCTTGACACAGCTG 3' | 94°C 30 sec, 54°C 30 sec and 72°C 40 sec |
| | GAACTCTTGCAGGTAATCCAGG | |
| IL-10 | 5' CCAGTTTACCTGGTAGAAGTGATG 3' | 94°C 30 sec, 56°C 30 sec and 72°C 40 sec |
| | TGTCTAGGTCCTGGAGTCCAGCAGACTCAA | |

The results of electrophoresis testing of the PCR product shown in Figure 11 indicate that when mice are vaccinated with the T-cell immune response inhibitor formed of the nucleic acid vaccine pcD-VP1 and pcD-VP1 expression protein antigen VP1 or the T-cell immune response inhibitor formed of bovine foot and mouth disease inactivated O-type vaccine (purchased from the Lanzhou Veterinary Medicine Research Institute) and pcD-VP1 (groups three, four, five, seven, eight and nine), the animal's in vivo IL-4 and IL-10 increase and its IL-2, IFN-γ levels decrease. The explanation is that in animals vaccinated with a T-cell immune response inhibitor formed of a targeted pathogen nucleic acid vaccine and the expression protein antigen for said nucleic acid vaccine, and vaccinated with a T-cell immune response inhibitor formed of the inactivated pathogen and the nucleic acid vaccine for said targeted pathogen's expression proteins, it elicits initial immunosuppression activity of cellular interleukin of IL-4 and IL-10 and proves that the compound completely suppresses T-cell activity through IL-4 and IL-10. In Figure 11, the X-axis is immunity groups one through ten.

### Industrial applications

The present invention and its currently existing technology possess the following advantages:
1. The T-cell immune response inhibitor in the present invention, compared to chemical medications such as Prograf (FK506), cyclosporin A (CsA), mycophenolate mofetil (MMF), azathioprine (Aza), prednisone (Pred), methylprednisolone (MP) and antibodies such as
   OKT4, is safer and has better selective suppression of the organism's T-cell immune response, thus it may effectively be applied to treatment of autoimmune disease, organ transplants and other arenas for controlling T-cell levels.
2. The T-cell immune response inhibitor in the present invention may stimulate the organism to produce the normal specific antibody immune response and inhibit the specific cellular immune response, especially the Th1 immune response. Said specific cellular immune response is mediated through enhancement of interleukin 10 levels and suppression of interferon IFN-γ levels. Enhanced interleukin 10 levels regulate the strengthened response of the organism's immune system through effective regulatory functioning and are an important means to keep the organism from suffering unnecessary loss of immunity. Therefore the T-cell immunity response inhibitor in the present invention is able to specifically inhibit the specific pathogen to induce loss of immunity and effectively overcome the inadequacies of nonspecific immuno-suppression.
3. The T-cell immune response inhibitor in the present invention does not require special response conditions. It may be manufactured using the equipment in general biological and pharmaceutical factories, its production methods are simple and production is easily industrialized.
4. The T-cell immune response inhibitor in the present invention may be used to treat the following autoimmune diseases: systemic lupus erythematosus (SLE), rheumatoid arthritis (RA), chronic lymphatic (Hashimoto's) thyroiditis, toxic goiter (Grave's disease), polyarteritis nodosa, insulin-dependent diabetes mellitus, myasthenia gravis, chronic active hepatitis, chronic ulcerative colitis, pernicious anemia with chronic atrophic gastritis, allergic encephalomyelitis, Goodpasture's syndrome, scleroderma, common pemphigus, pemphigoid, adrenocortical insufficiency, primary biliary cirrhosis of the liver, multiple sclerosis, acute polyneuroradiculitis and other serious autoimmune diseases; and it may be used to suppress the autoimmune rejection response in organ transplants.
5. The T-cell immune response inhibitor in the present invention may be used to treat allergic reactions caused by the following frequently seen allergens: dust mites, fleas, cockroaches, animal fur, pollen, mold, bacteria, virus- and tobacco smoke-induced skin and respiratory tract injuries, and the occurrence of allergic response or immunity overstimulation- induced allergic immune disorders: contact dermatitis, urticaria, allergic rhinitis, asthma, nephritis, hyperthyroidism, viral hepatitis immuno-hypersensitivity, etc.

## Claims

1. A T-cell immune response inhibitor that comprises a targeted pathogen nucleic acid vaccine and said nucleic acid vaccine's expression protein antigen; or a targeted pathogen nucleic acid vaccine and said nucleic acid vaccine's expression protein antigen's active polypeptide; or an inactivated pathogen and said targeted pathogen's nucleic acid vaccine.

2. A T-cell immune response inhibitor according to Patent Claim 1, wherein said T-cell immune response inhibitor comprises a single package or a mixture of the targeted pathogen nucleic acid vaccine and said nucleic acid vaccine's expression protein antigen.

3. A T-cell immune response inhibitor according to Patent Claim 2, wherein the physical proportion of said T-cell immune response inhibitor's targeted pathogen nucleic acid vaccine and said nucleic acid vaccine's expression protein antigen is 2:1 to 10:1.

4. A T-cell immune response inhibitor according to Patent Claim 3, wherein the physical proportion of said T-cell immune response inhibitor's targeted pathogen nucleic acid vaccine and said nucleic acid vaccine's expression protein antigen is 5:1.

5. A T-cell immune response inhibitor according to Patent Claim 1, wherein said T-cell immune response inhibitor comprises a single package or a mixture of the targeted pathogen nucleic acid vaccine and said nucleic acid vaccine's expression protein antigen's active polypeptide.

6. A T-cell immune response inhibitor according to Patent Claim 5, wherein the physical proportion of said T-cell immune response inhibitor's targeted pathogen nucleic acid vaccine and said nucleic acid vaccine's expression protein antigen's active peptide is 1:5 to 5:1.

7. A T-cell immune response inhibitor according to Patent Claim 1, wherein said T-cell immune response inhibitor comprises a single package or a mixture of the inactivated pathogen and the targeted pathogen nucleic acid vaccine.

8. A T-cell immune response inhibitor according to Patent Claim 7, wherein the physical proportion of the inactivated pathogen and the targeted pathogen nucleic acid vaccine in said T-cell immune response inhibitor is 1:2 to 1:10.

9. Any one of the T-cell immune response inhibitors according to Patent Claims 1 to 8, wherein said T-cell immune response inhibitor also comprises an immunological adjuvant.

10. Any one of the T-cell immune response inhibitors according to Patent Claims 1 to 8, wherein said nucleic acid vaccine is a eukaryote cell expression carrier containing a protein antigen encoded gene.

11. A T-cell immune response inhibitor according to Patent Claim 10, wherein the regulatory protein antigen encoded gene expression promoter in said eukaryote cell expression carrier is RSV, CMV and SV40 viral promoters.

12. A T-cell immune response inhibitor according to Patent Claim 10, wherein said eukaryote cell expression carrier is a plasmid expression carrier, virus or bacteriophage expression carrier, and the expression carrier is formed of plasmid DNA and viral or bacteriophage DNA or the expression carrier is formed of plasmid DNA and a chromosomal DNA fragment.

13. A T-cell immune response inhibitor according to Patent Claim 10, wherein said protein antigen encoded gene's DNA may be artificially synthesized or derived from double-stranded DNA obtained from microbes, eukaryote and plant cells or tissues.

14. A T-cell immune response inhibitor according to Patent Claims 2, 3 or 4, wherein said protein antigen is protein obtained through artificial synthesis or through biological production.

15. A T-cell immune response inhibitor according to Patent Claims 5 or 6, wherein said protein antigen's active polypeptides is obtained through artificial synthesis or biological production.

16. A T-cell immune response inhibitor according to Patent Claim 14, wherein the production of said biological organism may utilize enhanced production of Escherichia coli or bacillocin or saccharomycete or other eukaryote cellular organisms under artificial culture conditions.

17. A T-cell immune response inhibitor according to Patent Claim 15, wherein the production of said biological organism may utilize enhanced production of Escherichia coli or bacillocin or saccharomycete or other eukaryote cellular organisms under artificial culture conditions.

18. A T-cell immune response inhibitor according to Patent Claims 7 or 8, wherein said inactivated pathogen may use commonly known methods to separate and produce the virus, pathogenic bacteria, parasite and allergenic substance to obtain the noninfectious pathogen.

19. A T-cell immune response inhibitor according to Patent Claim 18, wherein said inactivated pathogen may be mixed directly with the nucleic acid vaccine or after emulsifying with mineral oil the inactivated pathogen may be mixed with the nucleic acid vaccine.
